# EUROPEAN PATENT APPLICATION

(11) **EP 4 545 652 A1**
(43) Date of publication of application: **30.04.2025**
(21) Application number: 23206348.7
(22) Date of filing: 27.10.2023
(51) Int. Cl.: C12Q 1/6883

(54) **METHOD TO PREDICT A NEURODEGENERATIVE DISEASE PROGRESSION**

(71) Applicant: IFOM - Istituto Fondazione di Oncologia Molecolare ETS, 20139 Milano (IT); Erasmus University Rotterdam Medical Center, 3015 GE Rotterdam (NL)
(72) Inventor: Mastroberardino, Pier Giorgio, 3021 XS Rotterdam (NL)
(74) Representative: Pace Napoleone, Maria

(57) **Abstract**

The present invention relates to a method of screening a patient affected by a neurodegenerative disease, such as Parkinson's disease, by determining the expression level of one or more RNA transcript in an isolated sample from said subject, wherein said RNA transcript is or comprises the RNA transcript of a gene coding for a product involved in at least one DNA repair process. Kits and computer programs for performing the method are also included.

## Description

### FIELD OF THE INVENTION

The present invention relates to the field of methods to predict the progression of a disease.

In particular, it is related to a method of classifying a patient affected by a neurodegenerative disease, such as Parkinson's disease, by determining the expression level of one or more mRNAs in an isolated sample from said subject.

### BACKGROUND OF THE INVENTION

Parkinson's disease (PD) is a common neurodegenerative disorder affecting principally, but not exclusively dopaminergic neurons in the nigrostriatal circuits. PD is largely idiopathic and less than 10% of cases can be attributed to monogenic mutations; the genetic forms, however, informed on the intricate pathogenic mechanisms underlying disease progression that include perturbation in protein homeostasis, oxido-reductive balance, mitochondrial function, and intracellular trafficking (Mullin and Schapira 2015, Poewe, Seppi et al. 2017). These alterations typically operate in a complex combination and converge to cause PD pathognomonic motor symptoms. The disease, however, is remarkably heterogeneous (Foltynie, Brayne et al. 2002) and patients display significant variability from the standpoint of both clinical presentation and disease progression rate, which is attributed to differences in the underlying pathophysiological processes (Berg, Borghammer et al. 2021). Consequently, PD has also been viewed as syndrome comprising several disease subtypes Rajput, Voll et al. 2009, Titova, Padmakumar et al. 2017.

Understanding of the causes of PD heterogeneity, however, is extremely rudimentary and stratification of patients in homogeneous groups based on the expected clinical presentation and disease progression remains at present unachievable.

This drawback represents a major confounding effect in clinical trials given that treatment of a heterogeneous cohort of patients will likely lead to equally heterogeneous outcomes (Kieburtz and Olanow 2015, Athauda and Foltynie 2016).

Additionally, stratification of patients in clinically homogeneous groups could favor the development of personalized, more effective treatments.

The screening of patients for stratification and development of personalized treatments is an ongoing need also in other neurodegenerative diseases, such as Alzheimer's disease (Zetterberg, 2021).

Therefore, there is still the need of a method to identify subjects with a neurodegenerative disease with faster disease progression.

In particular, there is still the need of a method to classify Parkinson's disease patients in homogeneous groups.

### SUMMARY OF THE INVENTION

It was now found the presence of a reduced expression of DNA repair pathways and a fingerprint of DNA damage accumulation in PD.

Data highlight a remarkable difference in the transcriptomic landscape of slow versus fast progressors and indicate that, in the latter group, the transcriptome drifts significantly.

It has also been now found that defective DNA repair pathways predict motor symptoms severity, therefore identifying patients with faster disease progression.

Patients' stratification is a matter of the utmost importance in the field of neurodegenerative diseases. Patients heterogeneity may in fact contribute to clinical trials failure given that experimental therapeutics will conceivably exert different effects depending upon the individual pathogenic mechanisms governing disease progression.

Advantageously, a screening based on the expression of DNA repair pathways can be easily implemented in the recruiting of patients in clinical trials in order to refine patients selection to reduce heterogeneity in cohorts and ultimately to design better, more informative clinical trials.

Also, the patient classification is advantageous to develop a personalized and more effective treatment.

Neurodegenerative diseases, such as Alzheimer's and Huntington's disease, share clinical similarities and common pathogenic mechanisms with PD, for instance proteotoxicity and mitochondrial dysfunction (see for example Prusiner, 2001). Therefore the above findings are applicable to various neurogenerative diseases.

It is an object of the invention an *in vitro* method of screening a subject suffering from a neurodegenerative disease or at risk of developing a neurodegenerative disease for predicting prognosis of the disease comprising at least the step of determining the expression level of at least one RNA transcript in an isolated biological sample from said subject, wherein said RNA transcript is or comprises the RNA transcript of a gene coding for a product involved in at least one DNA repair process.

Said neurodegenerative disease is preferably selected from the group consisting of: Parkinson's disease, Alzheimer's disease, Huntington's disease, Frontotemporal dementia, Pick's disease, progressive supranuclear palsy, amyotrophic lateral sclerosis, and spinocerebellar ataxias.

More preferably, said neurodegenerative disease is Parkinson's disease.

Said RNA transcript is preferably a mRNA.

Preferably, said gene codes for a product involved in one or more DNA repair processes selected from nucleotide excision repair, base excision repair, DNA synthesis, double strand break repair and homologous recombination.

More preferably, said gene is selected from the group consisting of:
ERCC8, ERCC3, XPA, DDB1, RBX1, POLE3, POLD2, RPA1, ATR, BARD1, MRE11, ERCC5, POLR2G, RPA2, TP53, POLR2I, POLR2J, POLR2E, GTF2H3, RFC4, GTF2H1, ERCC4, RFC3, MPG, CETN2, GTF2H5, POLD4, POLE4, POLR2K, POLR2D, POLL, POLR2H, ERCC2, RPA3, POLD1, POLR2F, and ERCC1.

In a preferred embodiment, said method comprises determining the expression level of RNA transcripts of at least 3 genes, preferably of from 3 to 37 genes. In this embodiment, an expression level pattern is obtained.

In a preferred embodiment, said gene is a gene coding for a product involved in nucleotide excision repair and it is selected from ERCC8, ERCC3, XPA, DDB1, and RBX1.

In a preferred embodiment, said gene is a gene coding for a product involved in the processes of base excision repair and DNA synthesis and it is selected from POLE3, POLD2 and RPA1.

In a preferred embodiment, said gene is a gene coding for a product involved in the processes of double strand break repair and homologous recombination and it is selected from ATR, BARD1 and MRE11.

In a preferred embodiment, the method comprises determining expression level of RNA transcripts of the following genes: ERCC8, ERCC3, XPA, DDB1, RBX1, POLE3, POLD2 , RPA1, ATR, BARD1 and MRE11.

In a more preferred embodiment, the method comprises determining expression level of RNA transcripts of the following genes: ERCC8, ERCC1 and RBX1.

The method of the invention preferably comprises a further step b) wherein the expression level of the RNA transcripts is normalized by comparison with the expression level of one or more reference RNA transcripts, or their protein translation products. Preferably, the method further comprises a step c) of determining a prognosis response by means of said comparison.

In an embodiment, determining a prognosis response includes classifying said subject in one of at least two classes corresponding to levels of progression of the disease. Preferably, said classes are two and corresponds to slow and fast progression of the disease, preferably Parkinson's disease.

It was also found that RNA transcripts coded by longer genes are significantly downregulated in PD patients.

It is therefore a further object of the invention an *in vitro* method of screening a subject suffering from a neurodegenerative disease, preferably Parkinson's disease, for predicting prognosis of the disease comprising at least the step of determining the expression level of at least one RNA transcript in an isolated biological sample from said subject, wherein said RNA transcript is coded by a gene longer or shorter than the median length of coding genes.

A kit for performing the method of the invention is a further object of the invention, said kit comprising at least means for determining in an isolated biological sample from a subject the expression level of at least one RNA transcript of a gene coding for a product involved in at least one DNA repair process and/or of at least one RNA transcript coded by a gene longer or shorter than the median length of coding genes. Said kit preferably further comprises means to normalize the expression level of the RNA transcripts by comparison with the expression level of one or more reference RNA transcripts, or their protein translation products, for example it comprises a reference pattern of RNA transcript expression levels.

The method of the invention can be implemented in a data processing device, such as a computer. Any suitable data processing device can be used for implementing the method of the invention.

A computer program comprising instructions which, when the program is executed by a computer, cause the computer to carry out the method of the invention is a further object of the invention.

A data processing device comprising means adapted for carrying out the method of the invention is a further object of the invention. Said data processing device preferably comprises:
i) means for receiving data representing the expression level of at least one RNA transcript in an isolated biological sample from a subject, wherein said RNA transcript is the RNA transcript of a gene coding for a product involved in at least one DNA repair process and/or a RNA transcript coded by a gene that is longer or shorter than the median length of coding genes;
ii) means for receiving data representing at least one reference pattern of RNA transcript expression levels;
iii) means for comparing said data received by means i) with said data received by means ii) and providing a prognosis of the disease.

The method of the invention can be advantageously carried out in an isolated sample from the patient, such as a blood sample, avoiding invasive procedures such as biopsies.

### DETAILED DESCRIPTION OF THE INVENTION

### Figures

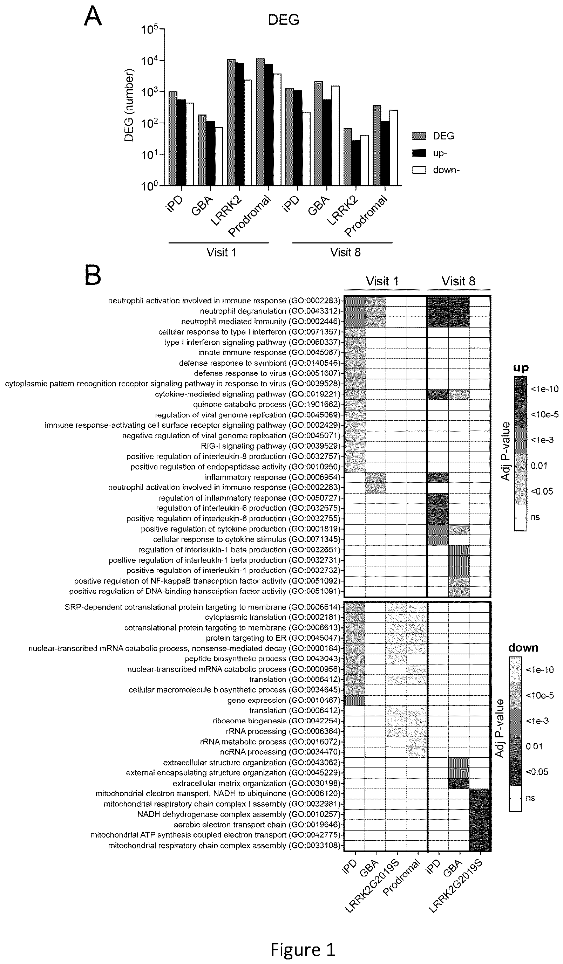

**Figure 1****. Over-representation analysis of significantly Differentially Expressed Gene DEG in PD patients' blood.** (**A**) Summary histogram of the number of significantly total, upand downregulated DEG in the analyzed experimental groups, at the intake visit and after 36 months (i.e. visit 8). (**B**) Over-represented Gene Ontology up- and down-regulated processes based on significantly DEG at visit 1 and 8. Gray tones indicate statistical significance, which is higher in lighter tones. White boxes indicate that the process is not altered. Upregulated processes are largely related to inflammatory processes, while the down-regulated ones predominantly concern macromolecular synthesis. The sensitivity of the analysis based on significantly DEG is modest and failed to detect alterations, for instance in GBA patients or prodromal cases at visit 1. In both (**A**) and (**B**) significance was set at adjusted P-value <0.05. Adjusted P-values were obtained using Fisher's exact test.

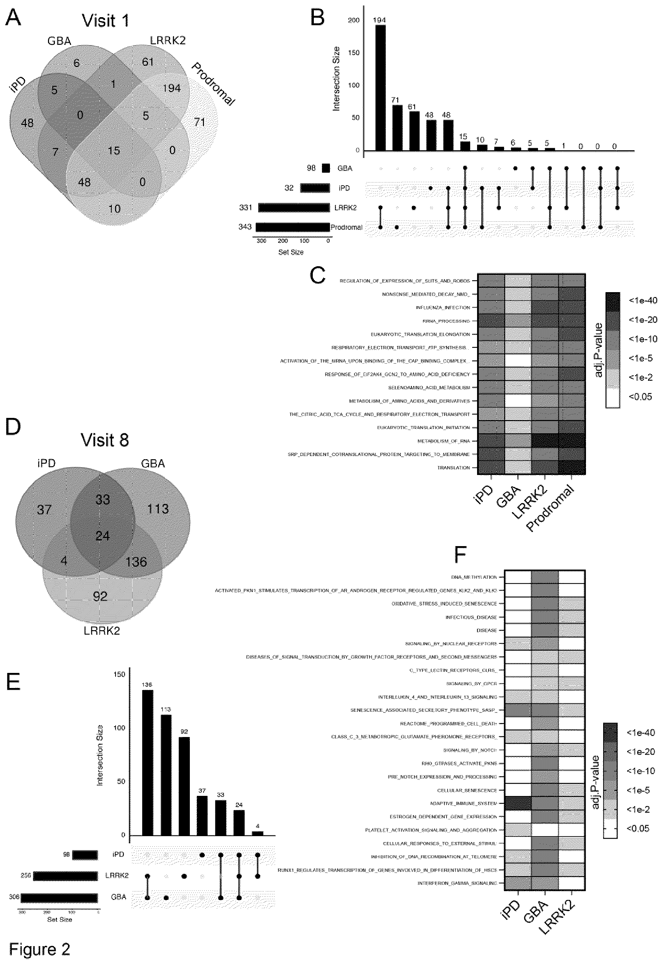

**Figure 2****. Differentially regulated pathways identified by gene set enrichment analysis (GSEA).** (**A**) Venn diagram and (**B**) UpSet plot of enriched GSEA Reactome pathways indicate that 15 are shared among the different analyzed groups. (**C**) The 15 common pathways at visit 1 include mechanisms related to macromolecular synthesis, mitochondrial function, and immunity. (**D**) Venn diagram and (**E**) UpSet plot of enriched GSEA Reactome pathways show 24 common pathways among the studied groups at visit 8. (**F**) Common pathways at visit 8 differ than those identified at visit 1 and include general disease related processes, senescence, and inflammation.

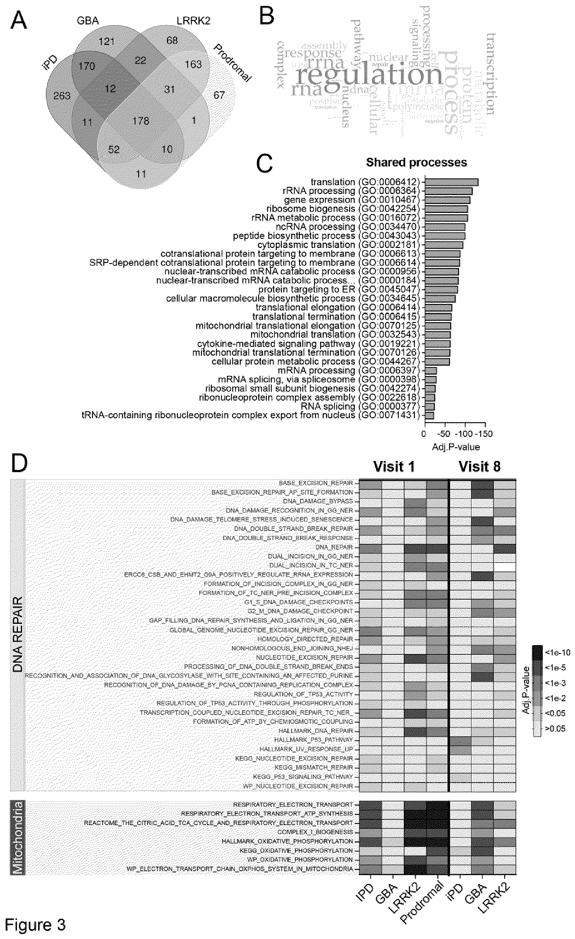

**Figure 3****. Leading edge gene analysis and GSEA identify alteration in processes related to macromolecular synthesis and DNA repair.** (**A**) Venn diagram based on the leading edge genes (LEG) identified by GSEA providing the most significant contribution to the top 20 deregulated pathways in the datasets. 178 genes are common to the studied experimental groups. (**B**) Text mining indicates that pathways related to the 178 LEG related to processes involving RNA, transcription, and polymerase activity, all of which are intertwined with DNA damage and repair. (**C**) Histogram showing the deregulated processes governed by the identified LEG. lea GSEA pro and C) The analysis revealed processes largely related to RNA metabolism. (**D**) Several DNA repair pathways are downregulated in PD, albeit at different time points. GBA patients, for instance, display alterations only at visit 8, while downregulation is prominent at visit 1 in iPD patients. As expected, genes related to mitochondrial function and complex I assembly are downregulated in PD.

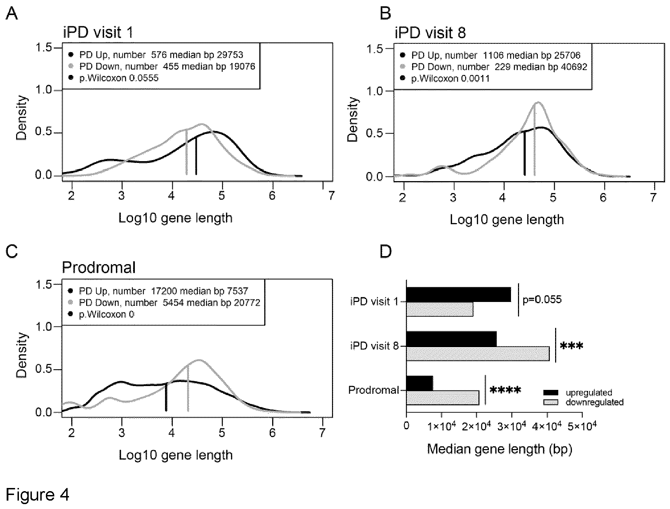

**Figure 4****. Biased reduction in the expression of longer genes in PD.** Frequency plots of gene length of up- and downregulated genes (red and green trace respectively) in iPD at visit 1 (**A**) and at visit 8 (**B**), and in prodromal cases (**C**). Histogram summarizing the findings in frequency plots showing that downredulated genes are significantly longer in iPD at visit 8 and in prodromal cases. P-value for iPD at visit 1 is close to significance (p=0.055). (**D**) Bar graph summarizing the effects Statistical significance of the distributions was tested with Wilcoxon signed-rank test (p.Wilcoxon, p<0.001 ***, p<0.0001 ****).

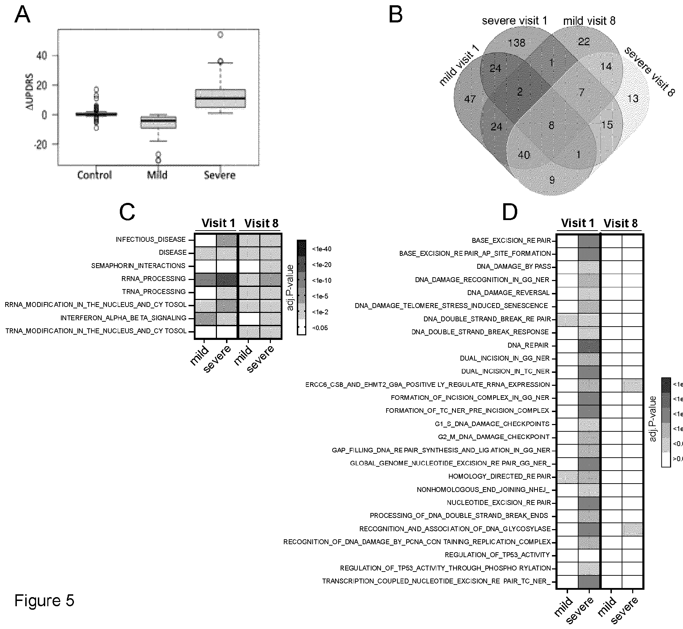

**Figure 5****. GSEA in patients with different disease progression.** (**A**) Summary boxplot of deltaUPDRS, defined as the difference in UPDRS score between visit 8 and visit 1 in iPD patients. GO-BP analysis at visit 1 in mild versus severe patients. (**B**) Venn Diagram of GSEA Reactome pathways detected 14 shared pathways between mild and severe cases at the two observed timepoints, and overall reveals differences between groups and time of analysis. (**C**) The 14 shared pathways relate to general processes of disease and to inflammation. (**D**) DNA repair pathways are predominantly altered in patients with severe progressions, at visit 1.

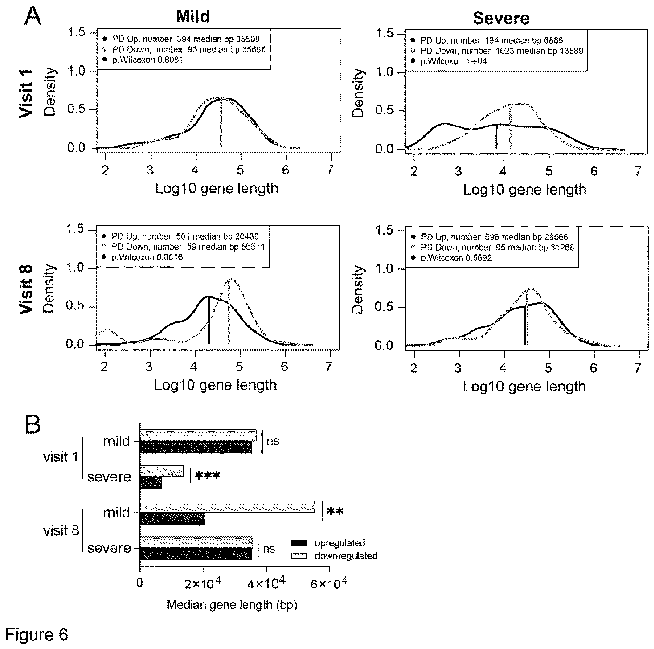

**Figure 6****. Biased reduction in the expression of longer genes in patients with different disease progression.** (**A**) Frequency plots of gene length of up- and downregulated genes (red and green trace respectively) in iPD with mild and severe progression at visit 1 and 8. (**B**) Bar graph summarizing the results shown in the frequency plots (p.Wilcoxon, p<0. 01, **, p<0.001, ***). Statistical significance of the distributions was tested with Wilcoxon signed-rank test. Gene length is in log10 scale.

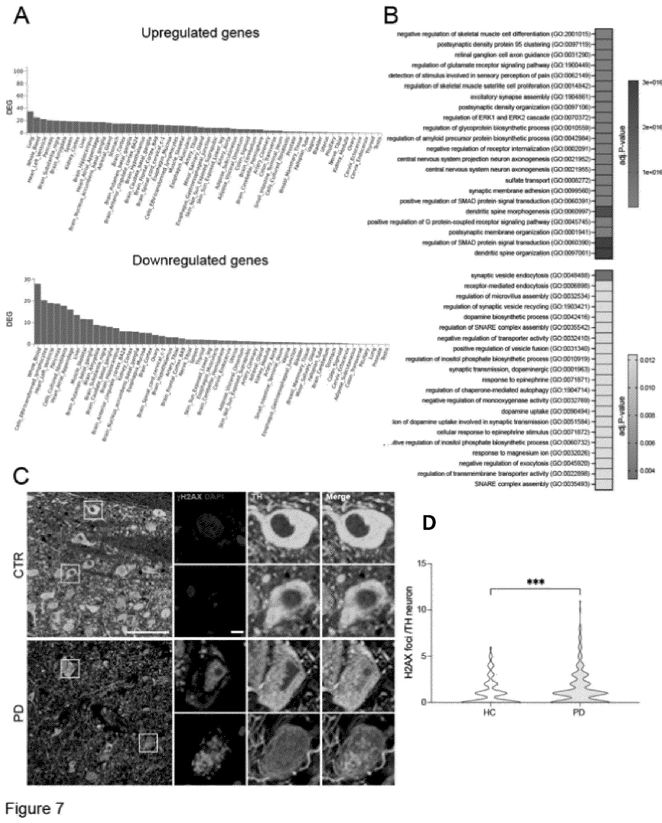

**Figure 7****. Functional mapping association analysis of statistically DEG in iPD samples.** (**A**) The analytical framework indicates that the molecular signature includes a strong component of both up- and downregulated transcripts that are typical of cells in the putamen and basal ganglia brain regions. (**B**) Histogram showing the most representative up- and down-regulated genes in iPD blood that are also typical of cells in the substantia nigra. (**C**) Representative immunohistochemistry images of tyrosine hydroxylase positive neurons substantia nigra pars compacta of iPD patients (PD N=9, healthy subjects N=7) displaying gamma-H2AX foci (insets). (**D**) Quantification of gamma-H2AX foci demonstrates increased signs 5 of DNA damage signs iPD brains (p<0.0001, two-sided unpaired Student's t-test).

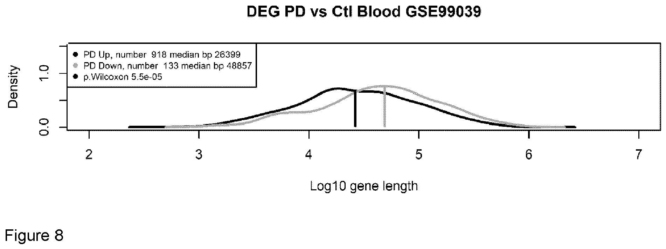

**Figure 8****.** Analysis for length-biased alterations in transcription output in additional datasets. Frequency plots of gene length in a different PD blood dataset (GSE99309) confirm biased suppression of longer versus shorter genes in PD specimens. In the context of the present invention, for "a product involved in at least one DNA repair process" is intended a product which takes part to a process related to DNA repair, for example a DNA repair protein.

In the context of the present invention, for "DNA repair process" is intended a process, such as a pathway, which is part of a collection of processes by which a cell identifies and corrects damage to the DNA molecules that encode its genome. Exemplary DNA repair processes are base excision repair (BER), nucleotide excision repair (NER), mismatch repair (MMR), homologous recombination (HR) and non-homologous end joining (NHEJ).

In the context of the present invention, for "RNA transcript" is intended a RNA product obtained by transcription of DNA. It includes primary transcript, mRNA, precursor mRNA (pre-mRNA), mature mRNA, rRNA and tRNA. For "mRNA" is intended "messenger ribonucleic acid".

In the context of the present invention, for "neurodegenerative disease" is intended a disease characterized by a progressive loss of structure and/or of function of neurons and/or characterized by neurodegeneration. Exemplary neurodegenerative diseases are Parkinson's disease, Alzheimer's disease, Huntington's disease, Frontotemporal dementia, Pick's disease, progressive supranuclear palsy, amyotrophic lateral sclerosis, and spinocerebellar ataxias. All such diseases are well known in the art.

In the context of the present invention, for "prognosis" is intended the likely or expected development of a disease, in particular the slow or fast progression of the disease. For "prognosis of Parkinson's disease" it is herein intended the probable progression of the disease, in particular the slow or fast progression of the disease.

In the context of the present invention, for "slow progression of Parkinson's disease" is intended an increment in the Unified Parkinson's Disease Rating Scale part III not greater than 1 scale unit in a three year period from the initial diagnosis.

In the context of the present invention, for "fast progression of Parkinson's disease" is intended an increment in the Unified Parkinson's Disease Rating Scale part III greater than 1 scale unit in a three year period from the initial diagnosis.

In the context of the present invention, for "subject at risk of developing a neurodegenerative disease" is intended an individual showing prodromal symptoms of neurodegeneration and/or prodromal symptoms of a neurodegenerative disease. In particular, for "subject at risk of developing Parkinson disease" is intended an individual displaying prodromal PD symptoms, for instance anosmia, REM sleep disorder and/or constipation, without manifesting any of PD cardinal symptoms.

In the context of the present invention, "gene" refers to a nucleic acid that is transcribed. In certain aspects, the gene includes regulatory sequences involved in transcription or message production. In particular embodiments, a gene comprises transcribed sequences that encode for a protein, polypeptide or peptide. As will be understood by those in the art, this functional term "gene" includes genomic sequences, RNA or cDNA sequences or smaller engineered nucleic acid segments, including 15 nucleic acid segments of a nontranscribed part of a gene, including but not limited to the nontranscribed promoter or enhancer regions of a gene. Smaller engineered nucleic acid segments may express, or may be adapted to express proteins, polypeptides, polypeptide domains, peptides, fusion proteins, mutant polypeptides and/or the like.

The biological sample can be a fluid sample or a tissue sample. Preferably it is a fluid sample, more preferably selected from a blood, plasma, saliva or urine sample. Preferably, it is a blood sample.

The RNA may be extracted from such sample and tested for the expression levels of the herein mentioned genes.

In a preferred aspect of the invention the method also comprises the extraction and/or purification of RNA from the biological sample.

In the method of the invention, determining the expression level of at least one RNA transcript can be carried out by one or more methods selected from a PCR-based methos, an array based method and a sequencing-based method. All such methods are known in the art and can be carried out according to the common general knowledge in the field.

The genes herein mentioned are preferably characterized by the sequences described by the following NCBI Gene ID (NCBI Database release version 257.0, August 15, 2023): ATR (Gene ID: 545), BARD1 (Gene ID: 580), CETN2 (Gene ID: 1069), DDB1 (Gene ID: 1642), ERCC1 (Gene ID: 2067), ERCC2 (Gene ID: 2068), ERCC3 (Gene ID: 2071), ERCC4 (Gene ID: 2072), ERCC5 (Gene ID: 2073), ERCC8 (Gene ID: 1161), GTF2H1 (Gene ID: 2965), GTF2H3 (Gene ID: 2967), GTF2H5 (Gene ID: 404672), MPG (Gene ID: 4350), MRE11 (Gene ID: 4361), POLD1 (Gene ID: 5424), POLD2 (Gene ID: 5425), POLD4 (Gene ID: 57804), POLE3 (Gene ID: 54107), POLE4 (Gene ID: 56655), POLL (Gene ID: 27343), POLR2D (Gene ID: 5433), POLR2E (Gene ID: 5434), POLR2F (Gene ID: 5435), POLR2G (Gene ID: 5436), POLR2H (Gene ID: 5437), POLR2I (Gene ID: 5438), POLR2J (Gene ID: 5439), POLR2K (Gene ID: 5440), RBX1 (Gene ID: 9978), RFC3 (Gene ID: 5983), RFC4 (Gene ID: 5984), RPA1 (Gene ID: 6117), RPA2 (Gene ID: 6118), RPA3 (Gene ID: 6119), TP53 (Gene ID: 7157), XPA (Gene ID: 7507).

The kit may for example comprise primers for polymerase chain reaction of one or more RNA transcripts of above mentioned genes.

In an exemplary embodiment of the invention, a sample is obtained from a subject affected by Parkinson's disease. The subject may be at any stage of the disease. The subject can be a patient with idiopathic PD, a patient carrying a PD genetic mutation, for example in LRRK2 or GBA genes, or a prodromal patient, i.e. a patient which do not present motor sign, but has a higher risk of developing PD. The sample can for example be obtained by blood sampling. The sample may be prepared for the subsequent RNA extraction.

Determining the relative level of expression of the genes herein mentioned in the biological sample may comprise determining the relative number of RNA transcripts, particularly mRNA transcripts, in the sample and/or determining the relative level of the corresponding protein in the sample. In specific embodiments, the relative level of protein in the sample is determined by an immunoassay whereby an antibody that binds the corresponding protein is contacted with the sample. The relative expression level of the genes is conveniently determined with respect to one or more standards. The standards may comprise, for example, a relative expression level compared to a control gene in the sample, such as one or more housekeeping genes, a zero expression level on the one hand and the expression level in a biological sample, such as a blood sample, of a normal control group on the other hand. The standard may also comprise the expression level in a standard cell line.

Methods of determining the level of (m)RNA transcripts of a particular gene in a sample are well-known to those skilled in the art.

In particular embodiments of the invention, the size of the change in the expression level of the RNA transcripts in comparison to normal expression levels is indicative of the prognosis of the disease, in particular of Parkinson's disease. For "normal expression levels" is intended expression levels in a subject not affected by the disease. In some embodiments, expression of one or more of genes involved in DNA repair, in particular the herein disclosed genes, is decreased in individuals carachterized by a severe disease and/or a fast progression of the Parkinson's disease

According to an embodiment of the invention, the method allows to classify the subject in ore or more classes corresponding to different degrees of severity of Parkinson's disease. For example, the lower is the expression of the involved genes the more severe is the disease.

According to a further object of the invention, an *in vitro* method of screening a subject suffering from a neurodegenerative disease, preferably Parkinson's disease, for predicting prognosis of the disease comprises the step of determining the expression level of at least one RNA transcript in an isolated biological sample from said subject, wherein said RNA transcript is coded by a gene longer or shorter than the median length of coding genes.

In some embodiment, said method further comprises a step b) wherein the expression level of the RNA transcripts is normalized by comparison with the expression level of one or more reference RNA transcripts, or their protein translation products and a step c) of determining a prognosis response by means of said comparison. In some embodiments, a decrease of expression levels of RNA transcripts which are coded by genes longer than the median length of coding genes is indicative of a severe prognosis of the Parkinson's disease. For "severe prognosis" is intended an increment in the Unified Parkinson's Disease Rating Scale part III greater than 1 scale unit in a three year period from the initial diagnosis.

The invention will be now illustrated by the following examples.

### EXAMPLES

### Materials and methods

### Data and method

Blood transcriptome data from PPMI (Parkinson progressive markers initiative cohort) were downloaded from the Parkinson's Progression Markers Iniziative website (www.ppmiinfo.org). Briefly, data were processed in the following steps: the whole-transcriptome RNAseq was obtained starting from 1 ug aliquots of RNA isolated from PaxGene tubes. Data RNA was sequenced at Hudson Alpha's Genomic Services Lab on an Illumina NovaSeq6000. The samples were prepared using the NEB/Kapa (NEBKAP) based library prep. BCL's were converted to using bcltofastq v1.8.4, and FASTQ's were merged and aligned to GRCh38p12by STAR (v2.6.1d) on GENCODE v29. The IR3 (B38) (Phase1 and Phase2 count) was used for this analysis.

### Transcriptomic data preprocessing, quality control and batch correction

Downloaded salmon files were imported into R using Tximport. Only transcripts with at least 10 reads in total were retained for analysis. Raw read counts were supplied to DESeq2, which was used to perform a differential expression analysis across PD groups and controls. Rlog transformation was applied to the normalized counts to improve the distances/clustering for the PCA. To identify low quality samples, we performed visual inspection of the distribution of the reads.

### Identification of differentially expressed genes (DEGs)

We focused our investigations on two time points, that is at baseline (i.e. very first visit) and at visit 8 (i.e. 36 months after the first visit). Differential expressed genes (DEG) were estimated using log2 fold-change, the Wald test, and FDR p-value correction calculated as implemented in DESeq2. A gene was defined as differentially expressed (DEG) when its FDR was lower than 0.05.

### Identification of genetic variants in PD patients

Subjects with PD and controls and known genetic mutations associated with PD, such as GBA, LRRK2, and SNCA were enrolled in the genetic cohort and all the information can be found on the website of Parkinson Progression Markers Initiative. For this study we selected only subjects with a diagnosis of Parkinson's disease and non-affected control subjects, with and without mutations in LRRK2_{G2019S} (LRRK2 patients) or in the GBA gene with the following mutations: GBAN370S, GBAT408M, GBAE365K, GBAIVS2, GBA84GG or GBAL444P (GBA patients).

### Pathway Analysis

Pathway enrichment analysis was conducted using two different approaches, overrepresentation analysis (ORA) (Boyle, Weng et al. 2004) and gene set enrichment analysis (GSEA) (Subramanian, Tamayo et al. 2005). ORA focuses on significantly DEG (FDR ≤ 0.05) to inform whether these genes overrepresent known biological functions or processes. Because ORA processes only significantly DEG, it returns information based on relatively large differences. Functional differences, however, could stem from small, coordinated changes in group of related genes, i.e. expression modules, which by construction cannot be detected by a method bound to significantly DEG. GSEA addresses this limitation by analyzing all the genes ranked according the differences in expression between two groups.

ORA was performed via the EnrichR web tool using Gene Ontology 2018 (GO) for biological processes (Chen, Tan et al. 2013, Kuleshov, Jones et al. 2016, Xie, Bailey et al. 2021) from the pre-filtered list of differentially expressed genes obtained as described in the previous section. Fisher exact test was performed to determine the likelihood of obtaining at least the equivalent numbers of genes by as actually overlap between the input gene set and the genes present in each identified pathway.

GSEA was conducted on an unfiltered, ranked list of genes. Genes in each PD group compared with controls were ranked by the level of differential expression using a signal to noise metric, and a weighted enrichment statistic. Statistical significance of pathway enrichment score was ascertained by permutation testing over size matched random gene sets. Multiple testing was controlled by false positives with family-wise error rate (FWER) threshold of 5% (Subramanian, Tamayo et al. 2005), which is statistically more conservative than FDR.

We used GSEA v.4.2.2 that also includes the Kyoto Encyclopedia of Genes and Genomes (KEGG), Reactome Pathway Databases, Hallmark Gene Set Collection and WikiPathways (http://www.gsea-msigdb.org/gsea/msigdb/collections.jsp).

Pathway information was obtained from the Kyoto Encyclopedia of Genes and Genomes (KEGG) available at the Molecular Signatures Database (http://www.broadinstitute.org/gsea/msigdb/index.jsp) or from the Hallmark Gene Set Collection (http://www.gsea-msigdb.org/gsea/msigdb/collections.jsp).

### Gene Length analysis

BiomaRt (Smedley, Haider et al. 2009) was used to retrieve the gene length (exons and introns) of all genes in the human genome. Each list of DEGs was divided in downregulated and upregulated genes. The distribution of the log10 length of up and down were evaluated by normality with a Shapiro-Wilk test. Because all the distributions were not normal, a Mann-Whitney Wilcoxon test for non-paired samples test was used to evaluate whether the distributions of gene lengths of up and down regulated DEGs were different between the different comparisons. Additionally, a relative frequency (kernel density) plot of gene length and probability density for DEG in each comparison was drawn using the density function implemented in R. Kernel density estimates are related to histograms, but with the possibility to smooth and continuity by using a kernel function. The y axis represents the density probability for a specific range of values in the x axis.

### Delta UPDRS classification of patients

Delta UPDRS was calculated subtracting UPDRS III (MDS-Unified Parkinson's Disease Rating Scale) of last visit to the one of the first visit.

### Gene expression analysis with FUMA

FUMA is an online tool which identified significantly up- or downregulated differentially expressed gene sets across human tissue types with a Bonferroni corrected P < 0.05. GENE2FUNC, a tool of FUMA (https://fuma.ctglab.nl/) was run using GTex v8:tissue types and general tissue types (K. Watanabe, et al. 2017).

An enrichment analysis of differentially tissue expressed genes (DEG) was carried put. The direction of expression was considered with <0,1. Tissue specificity is tested using hypergeometric tests. FUMA reports gene sets with adjusted P-value ≤ 0.05 and the number of genes that overlap with the gene set > 1 by default. Data are reported in a histogram.

### Immunostaining procedures

Formalin fixed paraffin embedded fully anonymous human midbrain tissue sections derived from PD patients and age-matched healthy controls were kindly provided by the Queen Square Brain Bank for Neurological Disorders.

In order to minimize autofluorescence, sections have been treated for photobleaching as previously described (A. K. Meeker, et al. 2021).

Briefly, samples were incubated in alkaline hydrogen peroxide solution (25mL PBS, 4.5 mL H2O2 30%, 0.8mL NaOH 1M) in clear plastic petri dishes and exposed to white light by sandwiching the immersed slides between two light-emitting diode (LED) panels for 45' at 4C (A. K. Meeker, et al. 2021; Z. Du et al., 2019}. The procedure has been repeated twice, with full solution change between the processes. Sections were then rinsed in TBS, pH 7.5, containing 0.01% Tween-20 and 100 mM sucrose (TBS-Ts) and blocked for non-specific binding with 5% donkey serum in antibody dilution buffer (ADB - 2% BSA, threalose 1 mM in TBS). Anti -tyrosine Hydroxylase (TH - 1:500, MAB 318, Merk Millipore) and anti -gH2AX (1:500 - ab11174, Abcam) have been used diluted in ADB overnight (ON) at 4C. After washes in TBS-Ts, sections have been incubated with alexa-fluor antibodies (1:250 in ADB, ThermoFisher) and DAPI (1:3000 in TBS-Ts). Finally, prolong Glass anti-fade medium (P36980, ThermoFisher) has been used as mounting medium. Images were acquired with the Leica SP8 STED imaging acquisition system at 40X magnification and gammaH2AX foci count was performed in a semi-automatic way using FIJI/imageJ software.

### Results

### Example 1

### Parkinson's Disease Patients show dysregulation in DNA repair pathways

We analyzed datasets originated from patients examined at the intake visit (i.e. baseline or visit 1), and at a follow up visit after 36 months (i.e. visit 8). The number of DEG remarkably differed between experimental groups and time points (fig.1A), pointing to heterogeneity among different forms of PD.

To gather more insights on the molecular processes altered in PD and in the different studied forms, we performed pathway enrichment analysis using both Gene Ontology-Biological Processes (GO-BP) overrepresentation analysis (ORA) (Boyle, Weng et al. 2004) focusing initially on significantly DEG in the datasets. We note that because GO-BP processes only significantly DEG, the analysis returns information based on relatively pronounced differences. GO-BP confirmed the heterogeneity between PD forms and between the timepoints of observations. For instance, several upregulated pathways were unique for iPD at visit 1, others were unique for GBA or LRRK2 at visit 8 (fig.1B). The analysis also that upregulated processes largely relate to inflammation, consistently with previous studies (Craig, Hutchins et al. 2021), while downregulated processes mostly relate to macromolecular synthesis and mitochondrial biology. We found the latter evidence reassuring given the well-established involvement of mitochondria in PD etiopathogenesis. GO-BP based on significantly DEG, however, failed to identify significantly upregulated processes in LRRK2 and prodromal patients. Functional differences, however, could stem from small changes in groups of related genes that are coordinately expressed, i.e. expression modules, which cannot be detected by a method bound to significantly DEG. This limitation is addressed by gene set enrichment analysis (GSEA) (Subramanian, Tamayo et al. 2005), which uses all the ranked genes in the datasets, independently from individual statistical significance to calculate aggregated statistical significance in a set of related genes, i.e. a pathway.

GSEA against the database Reactome identified a total of 839 deregulated pathways in the four groups (i.e. iPD, GBA, LRRK2, and prodromal), at visit 1, with 471 unique elements. Only 15 pathways, however, were common to the four experimental groups (fig. 2A, B) and, although they included expected processes such as mitochondrial function, they predominantly related to macromolecular synthesis (fig. 2C). Interestingly, shared pathways at visit 8 were different than those identified at visit 1 and included processes related to immunity, cell death, disease, and senescence, which is a consequence of DNA damage accumulation (Von Zglinicki, Saretzki et al. 2005) (fig. 2D-F).

Alterations in macromolecular synthesis may be indicative of genome instability because DNA damage is intrinsically associated with transcription and therefore downstream translation (Lans, Hoeijmakers et al. 2019, Gyenis, Chang et al. 2023). Moreover, macromolecular synthesis has been hypothesized to be perturbed in PD (Martin 2016), despite extremely rudimentary understanding of their role in the disease, and previous transcriptome analysis revealed alterations in ribosome related pathways in PD patients' brains that are also detectable in DNA repair defective mice (Sepe, Milanese et al. 2016). To further confirm macromolecular synthesis perturbation in early symptomatic PD (i.e. visit 1), we performed transcriptome examination at a more granular level taking advantage of leading-edge gene (LEG) analysis. This approach circumvents the problem that several of the GSEA identified pathways contain the same genes, which could in turn lead to redundant and the analysis may therefore provide redundant results. GSEA identifies the subset of genes, i.e. LEG, that provide the highest contribution to the enrichment signal of a given pathway (Subramanian, Tamayo et al. 2005). LEG analysis therefore offers a more focused depiction of the transcriptomic changes by reducing the enriched gene sets dimensionality and ultimately facilitating the identification of crucial biological processes. We extracted the LEG contributing to the top 20 deregulated pathways in the datasets and asked which biological processes the 178 common genes (fig.3A) govern. The analysis revealed processes largely related to RNA metabolism (fig.3B, C), therefore confirming the results of the previous analysis and substantiating the rationale for investigating DNA repair in PD.

We then directly investigated whether DNA repair pathways are perturbed in PD and determined their up- or down regulation using GSEA. The analysis revealed downregulation of several Reactome DNA repair pathways at visit 1 in iPD, LRRK2, and prodromal cases (fig. 3D). Downregulated pathways included NER and the data are therefore consistent with our previous findings showing deranged NER capacity in PD iPD and LRRK2 patients (Sepe, Milanese et al. 2016). GBA patients, conversely, were relatively unaffected at visit 1, but displayed downregulation in many DNA pathways at visit 8. Interestingly, in the follow up visit alterations were remarkably reduced in iPD patients, and the only group that showed continued defects was that of LRRK2 cases. Because the outcome of GSEA may depend upon the specific database interrogated, we monitored DNA repair pathways using additional platforms, i.e., KEGG, Hallmarks, and Wikipathways, which confirmed our findings in Reactome (fig.3D). Importantly, the analysis also displayed downregulation of mitochondrial pathways (fig.3D), which is expected given the central role of dysfunction in these organelles in PD. Collectively these results demonstrate that DNA repair mechanisms are reduced in PD, albeit with different kinetics during disease progression among its different forms.

### Example 2

### Biased reduction of long genes transcription levels in PD points to DNA damage accumulation

DNA damage is considered as a stochastic event and it is therefore more likely to occur in longer than in shorter genes. Because DNA damage can block RNA polymerase progression and therefore transcription, its accumulation is paralleled by a biased reduction of longer genes' transcripts. Indeed, our and other laboratories demonstrated that analysis for length-biased alterations in transcription output (ALBATRO) mirrors DNA damage accumulation (Vermeij, Dolle et al. 2016, Milanese, Bombardieri et al. 2019, Gyenis, Chang et al. 2023).

ALBATRO could be calculated only for groups of DE genes greater than 400. In visit 1 specimens, ALBATRO did not reveal any biased reduction in longer gene transcripts when compared to healthy controls: longer genes were even more represented in PD specimens, but the test was not statistically significant (median length up-regulated genes 29753 bp, median length down-regulated genes 19076 bp, p.Wilcoxon=0.0555) (fig. 4A, D). Overrepresentation of longer genes may reflect an adaptive protective response given recent evidence indicating that longer transcripts are often related to anti-aging genes (Stoeger, Grant et al. 2022). At visit 8, however, ALBATRO did detect significant downregulation of longer-transcripts in PD specimens (median length up-regulated genes 25706 bp, median length down-regulated genes 40692 bp, p.Wilcoxon=0.0011) (fig. 4B, D). Interestingly, also prodromal cases showed biased downregulation of longer transcripts (fig.4C, D).

### Example 3

### Transcriptional deregulation of DNA repair pathways predicts disease severity.

Our results indicate that down-regulation of DNA repair pathways occurs early in the symptomatic stage of PD (i.e. is detectable at visit 1) and that surrogate measures of DNA damage accumulation (i.e. biased decline of long transcripts) increase with disease progression. We therefore asked whether deregulation of DNA repair pathways may inform on the rate of disease progression. We therefore stratified iPD patients examined at both visit 1 and visit 8 in two groups with different progression rate. One group was composed of patients with increased severity as measured by the UPDRS III score (severe group, deltaUPDRS=UPDRS(visit 8)-UPDRS(visit 1)>0, n=226). Another group was composed of patients who did not display worsening of the UPDRS III score (mild group, deltaUPDRS≤1, n=112). We compared these groups to healthy controls (n=152) (fig.5A). GSEA revealed again substantial heterogeneity and detected only 8 shared pathways between mild and severe cases at the two observed timepoints (fig.5B), which, again, predominantly described processes inherent to RNA metabolism. Other pathways related to disease and inflammation in general terms (fig. 5C).

When we investigated the expression of DNA repair pathways in these experimental groups, we found significant downregulation of many varied mechanisms, only at visit 1, in those patients that displayed deterioration of motor symptoms in follow up visit 8, 36 months later (fig.5D). Consistently with the analysis in unstratified iPD (fig. 3D), we did not detect significant alterations in DNA repair pathways at visit 8.

At visit 1, ALBATRO detected reduced transcription of longer genes only in severe cases; conversely, only mild cases displayed biased transcription reduction at visit 8 (fig.6A, B). Collectively, these elements indicate that measures of defective DNA repair and nuclear DNA damage accumulation may predict severity of disease progression and suggest that different kinetics govern these processes during the symptomatic phase of the disease.

### Example 4

### Findings in peripheral cells mirror alterations in PD brains

We next sought to gather evidence that may substantiate the relevance of our findings in peripheral blood cells for the pathophysiology of the central nervous system. We took advantage of a bioinformatic workflow to unravel associations with expression profile traits and cells specificity, i.e. Functional Mapping and Association analysis (FUMA) (Watanabe, Taskesen et al. 2017, Rodriguez-Fontenla and Carracedo 2021). In essence, the tool disentangles the complexity of a transcriptome originated from a pooled analysis, which includes a multitude of cell types, comparing these results with expression patterns originated at single cell level. This contrast therefore informs on the representation of a cellspecific molecular signature in a pooled dataset ultimately indicating a cross-tissue imputation to DEG. When interrogated on tissue specific molecular signatures, FUMA analysis indicated that the most prominent contribution to the profile of DEG is given by the whole blood signature. Of note, the analysis also highlighted the contribution of several brain regions, particularly the *putamen* and the basal ganglia (fig. 7A). Central Nervous System more represented genes were subject to GO over-representation analysis, which highlighted the involvement of processes relevant for PD that included dopamine uptake and biosynthesis, and dopamine-mediated neurotransmission (fig. 7B). These results support that the alterations in DNA damage and repair we documented in blood of PD patients may parallel defects at the level of the brain. To provide conclusive evidence on the involvement of DNA damage and repair in PD-related neurodegeneration, we performed immunohistochemical (IHC) detection of two established markers for DNA damage markers, i.e. gammaH2AX and 53BP1, in PD patients post-mortem tissues (N=8). IHC in the substantia nigra pars compacta reveals a significant increase in both markers as well as their colocalization in tyrosine hydroxylase positive neurons when compared to age- and sex-matched controls (fig. 7C). These findings are fully consistent with the evidence we gained at bioinformatic level and strongly implicate defective DNA repair and DNA damage accumulation in PD pathogenesis.

We also applied ALBATRO to an openly available dataset (GSE68719) characterizing the transcriptome of the substantia nigra of 29 PD patients against 44 neurologically normal controls. The analysis revealed biased reduction in longer transcripts levels (fig. 8), therefore confirming the relevance of our findings in blood for the brain.

### Discussion

Aging is PD principal risk factor and progressive accumulation of nuclear DNA damage is a causative hallmark of aging (Hoeijmakers 2009). In this study we performed a blood transcriptome bioinformatic analysis in the state-of-the-art longitudinal PPMI cohort.

Our results highlighted reduced expression of DNA repair pathways and a fingerprint of DNA damage accumulation in PD. DNA repair derangement, however, was mainly detected in those patients that displayed faster progression rate in the three years period. Reduced expression of DNA repair pathways preceded biased reduction of long gene expression, a reliable surrogate of DNA damage accumulation (Vermeij, Dolle et al. 2016, Milanese, Bombardieri et al. 2019, Gyenis, Chang et al. 2023). Of note, the use surrogate measures for DNA damage is necessary because detection of DNA chemical modifications is notoriously insidious given the large variety of possible chemical modifications, their very rapid kinetics, and the different consequences they may exert on cell function (Lindahl and Barnes 2000, Shrivastav, Li et al. 2009). These factors may lead to artefactual measures and consequently significant attention has been devoted to the use of alternative systems, for instance based on measures of repair efficiency (Feuerhahn and Egly 2008). Biased reduction of long gene expression circumvents these limitations providing a measure of age-related DNA damage accumulation (Gyenis, Chang et al. 2023).

Our data highlight a remarkable difference in the transcriptomic landscape of slow versus fast progressors and indicate that, in the latter group, the transcriptome drifts significantly in the three-year period of observation. It is tempting to speculate reduced genome maintenance, which is observed in fast progressing patients, contributes to the evolution of the transcriptome. This scenario would parallel that observed in cancer (Ben-David, Siranosian et al. 2018), even though genetic and transcriptional evolution in PD would be conceivably slower. Indeed, studying mouse models of genome instability we found that mild, rather than strong DNA repair defects elicit a dopaminergic phenotype recapitulating PD, also because of a pronounced adaptive, antioxidant response elicited by severe transcription stalling DNA damage (Sepe, Milanese et al. 2016, Milanese, Bombardieri et al. 2019).

We did not detect any alterations related to DNA repair pathways in GBA and not significant in LRRK2 mutants at visit 1. It might have been significant at visit 8 if the statistical power of this cohort would have been greater. The absence of alterations related to DNA repair in the GBA cohort, instead, point to different pathogenic mechanisms in this specific group of PD patients. This is consistent with reported differences in the presentation and age of onset of PD GBA carriers (Gegg, Menozzi et al. 2022).

Patients' stratification is a matter of the utmost importance in the field of neurodegenerative diseases (Wilson, Cookson et al. 2023). Patients heterogeneity may in fact contribute to clinical trials failure given that experimental therapeutics will conceivably exert different effects depending upon the individual pathogenic mechanisms governing disease progression. We provide evidence that, in the longitudinal cohort we analyzed, defective DNA repair pathways predict motor symptoms severity, therefore identifying patients with faster disease progression. This screening could be easily implemented while recruiting patients in future trials. The observation could be therefore relevant to refine patients selection to reduce heterogeneity in cohorts and ultimately to design better, more informative clinical trials (Rajput, Voll et al. 2009, Athauda and Foltynie 2016).

Defects in genome maintenance have been hypothesized, investigated, and detected in several neurodegenerative disorders (Jeppesen, Bohr et al. 2011, Sepe, Payan-Gomez et al. 2013, McKinnon 2017). Evidence gained in PD, in patients, is however limited (Gonzalez-Hunt and Sanders 2021), particularly at the level of large longitudinal cohorts. Here, we substantiate an involvement of age-related DNA damage and repair in PD.These findings, obtained at peripheral level, are also relevant for the central nervous system. FUMA analysis, in fact, revealed that the list of DEG is significantly contributed by transcripts that are critical for brain functioning and neuropathological analysis in post-mortem brains detected increased markers of DNA damage in PD. Overall, this study substantiates the concept that peripheral tissues may be informative of central pathology and is in agreement with evidence showing that risk loci for PD impact a wide spectrum of cellular processes, not necessarily related to the brain, to one of its anatomical areas, or to a specific cell type (Reynolds, Botía et al. 2019).

DNA sits at the hierarchical apex of biological information and corruption of its fidelity has broad impacts. DNA damage accumulation has been in fact associated with key mechanisms of neurodegeneration such as mitochondrial defects, oxidant stress, proteotoxic stress, and inflammation (Rodier, Coppe et al. 2009, Fang, Scheibye-Knudsen et al. 2016, Paull 2021, Pezone, Olivieri et al. 2023, Zhao, Simon et al. 2023). It is therefore plausible that, in PD, defective DNA maintenance connects with other better characterized pathogenic mechanisms. For instance, reduced capacity in the nucleotide excision repair sensitizes to MPTP and triggers alpha-synuclein stress, and LRRK2 biology impacts mitochondrial DNA quality (Sanders, Laganiere et al. 2014, Sepe, Milanese et al. 2016).

The main scope of this study was to identify a fingerprint of altered DNA maintenance in the PPMI cohort. Alterations were detected in patients that exhibited faster progression of motor symptoms in the 36-month period of observation. Defects in DNA repair may therefore constitute a predictive criterion for the progression rate of the disease. The analyses also revealed differences in the transcriptional landscape between slow and fast progressing patients, informing on different pathogenic mechanisms.

### References

Athauda, D. and T. Foltynie (2016). "Challenges in detecting disease modification in Parkinson's disease clinical trials." Parkinsonism & Related Disorders 32: 1-11.

Avila Cobos, F., J. Vandesompele, P. Mestdagh and K. De Preter (2018). "Computational deconvolution of transcriptomics data from mixed cell populations." Bioinformatics 34(11): 1969-1979.

Ben-David, U., B. Siranosian, G. Ha, H. Tang, Y. Oren, K. Hinohara, C. A. Strathdee, J. Dempster, N. J. Lyons and R. Burns (2018). "Genetic and transcriptional evolution alters cancer cell line drug response." Nature 560(7718): 325-330.

Berg, D., P. Borghammer, S.-M. Fereshtehnejad, S. Heinzel, J. Horsager, E. Schaeffer and R. B. Postuma (2021). "Prodromal Parkinson disease subtypes - key to understanding heterogeneity." Nature Reviews Neurology 17(6): 349-361.

Boyle, E. I., S. Weng, J. Gollub, H. Jin, D. Botstein, J. M. Cherry and G. Sherlock (2004). "GO::TermFinder-open source software for accessing Gene Ontology information and finding significantly enriched Gene Ontology terms associated with a list of genes." Bioinformatics 20(18): 3710-3715.

Chen, E. Y., C. M. Tan, Y. Kou, Q. Duan, Z. Wang, G. V. Meirelles, N. R. Clark and A. Ma'ayan (2013). "Enrichr: interactive and collaborative HTML5 gene list enrichment analysis tool." BMC Bioinformatics 14: 128.

Craig, D. W., E. Hutchins, I. Violich, E. Alsop, J. R. Gibbs, S. Levy, M. Robison, N. Prasad, T. Foroud, K. L. Crawford, A. W. Toga, T. G. Whitsett, S. Kim, B. Casey, A. Reimer, S. J. Hutten, M. Frasier, F. Kern, T. Fehlman, A. Keller, M. R. Cookson, K. Van Keuren-Jensen, S. Hutten, K. Van Keuren-Jensen and I. Parkinson Progression Marker (2021). "RNA sequencing of whole blood reveals early alterations in immune cells and gene expression in Parkinson's disease." Nature Aging 1(8): 734-747.

Z. Du et al., Qualifying antibodies for image-based immune profiling and multiplexed tissue imaging. Nat Protoc 14, 2900-2930 (2019)

Fang, E. F., M. Scheibye-Knudsen, K. F. Chua, M. P. Mattson, D. L. Croteau and V. A. Bohr (2016). "Nuclear DNA damage signalling to mitochondria in ageing." Nat Rev Mol Cell Biol 17(5): 308-321.

Feuerhahn, S. and J.-M. Egly (2008). "Tools to study DNA repair: what's in the box?" Trends in Genetics 24(9): 467-474.

Foltynie, T., C. Brayne and R. A. Barker (2002). "The heterogeneity of idiopathic Parkinson's disease." J Neurol 249(2): 138-145.

Gegg, M. E., E. Menozzi and A. H. V. Schapira (2022). "Glucocerebrosidase-associated Parkinson disease: Pathogenic mechanisms and potential drug treatments." Neurobiology of Disease 166: 105663.

Gonzalez-Hunt, C. P. and L. H. Sanders (2021). "DNA damage and repair in Parkinson's disease: Recent advances and new opportunities." J Neurosci Res 99(1): 180-189. Gyenis, A., J. Chang, J. Demmers, S. T. Bruens, S. Barnhoorn, R. M. C. Brandt, M. P. Baar, M. Raseta, K. W. J. Derks, J. H. J. Hoeijmakers and J. Pothof (2023). "Genome-wide RNA polymerase stalling shapes the transcriptome during aging." Nat Genet.

Hoeijmakers, J. H. (2009). "DNA damage, aging, and cancer." The New England journal of medicine 361(15): 1475-1485.

Jeppesen, D. K., V. A. Bohr and T. Stevnsner (2011). "DNA repair deficiency in neurodegeneration." Progress in Neurobiology 94(21550379): 166-200.

Kieburtz, K. and C. W. Olanow (2015). "Advances in clinical trials for movement disorders." Movement Disorders 30(11): 1580-1587.

Kuleshov, M. V., M. R. Jones, A. D. Rouillard, N. F. Fernandez, Q. Duan, Z. Wang, S. Koplev, S. L. Jenkins, K. M. Jagodnik, A. Lachmann, M. G. McDermott, C. D. Monteiro, G. W. Gundersen and A. Ma'ayan (2016). "Enrichr: a comprehensive gene set enrichment analysis web server 2016 update." Nucleic Acids Res 44(W1): W90-97.

Lans, H., J. H. J. Hoeijmakers, W. Vermeulen and J. A. Marteijn (2019). "The DNA damage Lindahl, T. and D. Barnes (2000). Repair of endogenous DNA damage. Cold Spring Harbor symposia on quantitative biology, Cold Spring Harbor Laboratory Press.

Martin, I. (2016). "Decoding Parkinson's Disease Pathogenesis: The Role of Deregulated mRNA Translation." J Parkinsons Dis 6(1): 17-27.

A. K. Meeker, C. M. Heaphy, C. M. Davis, S. Roy, E. A. Platz, Photochemical prebleaching of formalin-fixed archival prostate tissues significantly reduces autofluorescence to facilitate multiplex immunofluorescence staining. bioRxiv, 2021.2011.2009.467916 (2021)). McKinnon, P. J. (2017). "Genome integrity and disease prevention in the nervous system." Genes & development 31(12): 1180-1194.

Milanese, C., C. R. Bombardieri, S. Sepe, S. Barnhoorn, C. Payan-Gomez, D. Caruso, M. Audano, S. Pedretti, W. P. Vermeij, R. M. C. Brandt, A. Gyenis, M. M. Wamelink, A. S. de Wit, R. C. Janssens, R. Leen, A. B. P. van Kuilenburg, N. Mitro, J. H. J. Hoeijmakers and P. G. Mastroberardino (2019). "DNA damage and transcription stress cause ATP-mediated redesign of metabolism and potentiation of anti-oxidant buffering." Nat Commun 10(1): 4887.

Mullin, S. and A. H. Schapira (2015). "Pathogenic mechanisms of neurodegeneration in Parkinson disease." Neurologic Clinics 33(1): 1-17.

Paul, K. C., A. M. Binder, S. Horvath, C. Kusters, Q. Yan, I. D. Rosario, Y. Yu, J. Bronstein and B. Ritz (2021). "Accelerated hematopoietic mitotic aging measured by DNA methylation, blood cell lineage, and Parkinson's disease." BMC Genomics 22(1): 696.

Paull, T. T. (2021). "DNA damage and regulation of protein homeostasis." DNA Repair (Amst) 105: 103155.

Pezone, A., F. Olivieri, M. V. Napoli, A. Procopio, E. V. Avvedimento and A. Gabrielli (2023). "Inflammation and DNA damage: cause, effect or both." Nat Rev Rheumatol.

Pickrell, A. M. and R. J. Youle (2015). "The roles of PINK1, parkin, and mitochondrial fidelity in Parkinson's disease." Neuron 85(2): 257-273.

Poewe, W., K. Seppi, C. M. Tanner, G. M. Halliday, P. Brundin, J. Volkmann, A. E. Schrag and A. E. Lang (2017). "Parkinson disease." Nat Rev Dis Primers 3: 17013.

Prusiner, S.B., N Engl J Med (2001). " Neurodegenerative Diseases and Prions" 344:1516-1526.

Rajput, A. H., A. Voll, M. L. Rajput, C. A. Robinson and A. Rajput (2009). "Course in Parkinson disease subtypes." A 39-year clinicopathologic study 73(3): 206-212.

Reynolds, R. H., J. Botía, M. A. Nalls, A. J. Noyce, A. Nicolas, M. R. Cookson, S. Bandres-Ciga, J. R. Gibbs, D. G. Hernandez, A. B. Singleton, X. Reed, H. Leonard, C. Blauwendraat, F. Faghri, J. Bras, R. Guerreiro, A. Tucci, D. A. Kia, H. Houlden, H. Plun-Favreau, K. Y. Mok, N. W. Wood, R. Lovering, L. R'Bibo, M. Rizig, V. Chelban, D. Trabzuni, M. Tan, H. R. Morris, B. Middlehurst, J. Quinn, K. Billingsley, P. Holmans, K. J. Kinghorn, P. Lewis, V. Escott-Price, N. Williams, T. Foltynie, A. Brice, F. Danjou, S. Lesage, J.-C. Corvol, M. Martinez, A. Giri, C. Schulte, K. Brockmann, J. Simón-Sánchez, P. Heutink, T. Gasser, P. Rizzu, M. Sharma, J. M. Shulman, L. Robak, S. Lubbe, N. E. Mencacci, S. Finkbeiner, C. Lungu, S. W. Scholz, Z. Gan-Or, G. A. Rouleau, L. Krohan, J. J. van Hilten, J. Marinus, A. D. Adarmes-Gómez, I. Bernal-Bernal, M. Bonilla-Toribio, D. Buiza-Rueda, F. Carrillo, M. Carrión-Claro, P. Mir, P. Gómez-Garre, S. Jesús, M. A. Labrador-Espinosa, D. Macias, L. Vargas-González, C. Méndez-del-Barrio, T. Periñán-Tocino, C. Tejera-Parrado, M. Diez-Fairen, M. Aguilar, I. Alvarez, M. T. Boungiorno, M. Carcel, P. Pastor, J. P. Tartari, V. Alvarez, M. M. González, M. Blazquez, C. Garcia, E. Suarez-Sanmartin, F. J. Barrero, E. M. Rezola, J. A. B. Yarza, A. G. Pagola, A. L. de Munain Arregui, J. Ruiz-Martinez, D. Cerdan, J. Duarte, J. Clarimón, O. Dols-Icardo, J. Infante, J. Marín, J. Kulisevsky, J. Pagonabarraga, I. Gonzalez-Aramburu, A. S. Rodriguez, M. Sierra, R. Duran, C. Ruz, F. Vives, F. Escamilla-Sevilla, A. Mínguez, A. Cámara, Y. Compta, M. Ezquerra, M. J. Marti, M. Fernández, E. Muñoz, R. Fernández-Santiago, E. Tolosa, F. Valldeoriola, P. Garcia-Ruiz, M. J. G. Heredia, F. P. Errazquin, J. Hoenicka, A. Jimenez-Escrig, J. C. Martinez-Castrillo, J. L. Lopez-Sendon, I. M. Torres, C. Tabernero, L. Vela, A. Zimprich, L. Pihlstrom, S. Koks, P. Taba, K. Majamaa, A. Siitonen, N. U. Okubadejo, O. O. Ojo, T. Pitcher, T. Anderson, S. Bentley, J. Fowdar, G. Mellick, J. Dalrymple-Alford, A. K. Henders, I. Kassam, G. Montgomery, J. Sidorenko, F. Zhang, A. Xue, C. L. Vallerga, L. Wallace, N. R. Wray, J. Yang, P. M. Visscher, J. Gratten, P. A. Silburn, G. Halliday, I. Hickie, J. Kwok, S. Lewis, M. Kennedy, J. Pearson, J. Hardy, S. A. Gagliano Taliun, M. Ryten, C. International Parkinson's Disease Genomics and D. System Genomics of Parkinson's (2019). "Moving beyond neurons: the role of cell typespecific gene regulation in Parkinson's disease heritability." npj Parkinson's Disease 5(1): 6. Rodier, F., J. P. Coppe, C. K. Patil, W. A. Hoeijmakers, D. P. Munoz, S. R. Raza, A. Freund, E. Campeau, A. R. Davalos and J. Campisi (2009). "Persistent DNA damage signalling triggers senescence-associated inflammatory cytokine secretion." Nat Cell Biol 11(8): 973-979.

Rodriguez-Fontenla, C. and A. Carracedo (2021). "UTMOST, a single and cross-tissue TWAS (Transcriptome Wide Association Study), reveals new ASD (Autism Spectrum Disorder) associated genes." Translational Psychiatry 11(1): 256.

Sanders, L. H., J. Laganiere, O. Cooper, S. K. Mak, B. J. Vu, Y. A. Huang, D. E. Paschon, M. Vangipuram, R. Sundararajan, F. D. Urnov, J. W. Langston, P. D. Gregory, H. S. Zhang, J. T. Greenamyre, O. Isacson and B. Schule (2014). "LRRK2 mutations cause mitochondrial DNA damage in iPSC-derived neural cells from Parkinson's disease patients: reversal by gene correction." Neurobiol Dis 62: 381-386.

Sanders, L. H., J. McCoy, X. Hu, P. G. Mastroberardino, B. C. Dickinson, C. J. Chang, C. T. Chu, B. Van Houten and J. T. Greenamyre (2014). "Mitochondrial DNA damage: molecular marker of vulnerable nigral neurons in Parkinson's disease." Neurobiol Dis 70: 214-223. Sepe, S., C. Milanese, S. Gabriels, K. W. Derks, C. Payan-Gomez, I. W. F. van, Y. M. Rijksen, A. L. Nigg, S. Moreno, S. Cerri, F. Blandini, J. H. Hoeijmakers and P. G. Mastroberardino (2016). "Inefficient DNA Repair Is an Aging-Related Modifier of Parkinson's Disease." Cell Rep 15(9): 1866-1875.

Sepe, S., C. Payan-Gomez, C. Milanese, J. H. Hoeijmakers and P. G. Mastroberardino (2013). "Nucleotide excision repair in chronic neurodegenerative diseases." DNA repair. Shrivastav, N., D. Li and J. M. Essigmann (2009). "Chemical biology of mutagenesis and DNA repair: cellular responses to DNA alkylation." Carcinogenesis 31(1): 59-70.

Smedley, D., S. Haider, B. Ballester, R. Holland, D. London, G. Thorisson and A. Kasprzyk (2009). "BioMart--biological queries made easy." BMC Genomics 10: 22.

Smith, A. M., C. Depp, B. J. Ryan, G. I. Johnston, J. Alegre-Abarrategui, S. Evetts, M. Rolinski, F. Baig, C. Ruffmann, A. K. Simon, M. T. M. Hu and R. Wade-Martins (2018). "Mitochondrial dysfunction and increased glycolysis in prodromal and early Parkinson's blood cells." Mov Disord 33(10): 1580-1590.

Stoeger, T., R. A. Grant, A. C. McQuattie-Pimentel, K. R. Anekalla, S. S. Liu, H. Tejedor-Navarro, B. D. Singer, H. Abdala-Valencia, M. Schwake, M.-P. Tetreault, H. Perlman, W. E. Balch, N. S. Chandel, K. M. Ridge, J. I. Sznajder, R. I. Morimoto, A. V. Misharin, G. R. S. Budinger and L. A. Nunes Amaral (2022). "Aging is associated with a systemic lengthassociated transcriptome imbalance." Nature Aging 2(12): 1191-1206.

Subramanian, A., P. Tamayo, V. K. Mootha, S. Mukherjee, B. L. Ebert, M. A. Gillette, A. Paulovich, S. L. Pomeroy, T. R. Golub, E. S. Lander and J. P. Mesirov (2005). "Gene set enrichment analysis: a knowledge-based approach for interpreting genome-wide expression profiles." Proc Natl Acad Sci U S A 102(43): 15545-15550.

Titova, N., C. Padmakumar, S. J. G. Lewis and K. R. Chaudhuri (2017). "Parkinson's: a syndrome rather than a disease?" J Neural Transm (Vienna) 124(8): 907-914.

Vermeij, W. P., M. E. Dolle, E. Reiling, D. Jaarsma, C. Payan-Gomez, C. R. Bombardieri, H. Wu, A. J. Roks, S. M. Botter, B. C. van der Eerden, S. A. Youssef, R. V. Kuiper, B. Nagarajah, C. T. van Oostrom, R. M. Brandt, S. Barnhoorn, S. Imholz, J. L. Pennings, A. de Bruin, A. Gyenis, J. Pothof, J. Vijg, H. van Steeg and J. H. Hoeijmakers (2016). "Restricted diet delays accelerated ageing and genomic stress in DNA-repair-deficient mice." Nature 537(7620): 427-431.

Von Zglinicki, T., G. Saretzki, J. Ladhoff, F. d. A. di Fagagna and S. Jackson (2005). "Human cell senescence as a DNA damage response." Mechanisms of ageing and development 126(1): 111-117.

Watanabe, K., E. Taskesen, A. van Bochoven and D. Posthuma (2017). "Functional mapping and annotation of genetic associations with FUMA." Nature Communications 8(1): 1826.

Wilson, D. M., III, M. R. Cookson, L. Van Den Bosch, H. Zetterberg, D. M. Holtzman and I. Dewachter (2023). "Hallmarks of neurodegenerative diseases." Cell 186(4): 693-714.

Xie, Z., A. Bailey, M. V. Kuleshov, D. J. B. Clarke, J. E. Evangelista, S. L. Jenkins, A. Lachmann, M. L. Wojciechowicz, E. Kropiwnicki, K. M. Jagodnik, M. Jeon and A. Ma'ayan (2021). "Gene Set Knowledge Discovery with Enrichr." Current Protocols 1(3): e90.

Zetterbeg, H., Bendlin B. B. (2021). " Biomarkers for Alzheimer's disease - preparing for a new era of disease-modifying therapies "Mol Psychiatry 26(1): 296-308.

Zhao, Y., M. Simon, A. Seluanov and V. Gorbunova (2023). "DNA damage and repair in age-related inflammation." Nat Rev Immunol 23(2): 75-89.

## Claims

1. An *in vitro* method of screening a subject suffering from a neurodegenerative disease or at risk of developing a neurodegenerative disease for predicting prognosis of the disease comprising at least the step a) of determining the expression level of at least one RNA transcript in an isolated biological sample from said subject, wherein said RNA transcript is or comprises the RNA transcript of at least one gene coding for a product involved in at least one DNA repair process.

2. The method according to claim 1 wherein said RNA transcript is mRNA.

3. The method according to any of the preceding claims wherein said gene codes for a product involved in one or more DNA repair processes selected from nucleotide excision repair, base excision repair, DNA synthesis, double strand break repair and homologous recombination.

4. The method according to any of the preceding claims wherein said gene is selected from the group consisting of: ERCC8, ERCC3, XPA, DDB1, RBX1, POLE3, POLD2, RPA1, ATR, BARD1, MRE11, ERCC5, POLR2G, RPA2, TP53, POLR2I, POLR2J, POLR2E, GTF2H3, RFC4, GTF2H1, ERCC4, RFC3, MPG, CETN2, GTF2H5, POLD4, POLE4, POLR2K, POLR2D, POLL, POLR2H, ERCC2, RPA3, POLD1, POLR2F, and ERCC1.

5. The method according to any of the preceding claims wherein said method comprises determining the expression level of the RNA transcripts of at least 3 genes, preferably of from 3 to 37 genes.

6. The method according to any of the preceding claims wherein said gene codes for a product involved in nucleotide excision repair and is selected from ERCC8, ERCC3, XPA, DDB1, and RBX1 and/or said gene codes for a product involved in the processes of base excision repair and DNA synthesis and is selected from POLE3, POLD2 and RPA1 and/or said gene codes for a product involved in the processes of double strand break repair and homologous recombination and is selected from ATR, BARD1 and MRE11.

7. The method according to any of the preceding claims wherein the method comprises determining expression level of RNA transcripts of at least the following genes: ERCC8, ERCC1 and RBX1.

8. The method according to any of the preceding claims wherein it further comprises a step b) wherein the expression level of the at least one RNA transcript is normalized by comparison with the expression level of one or more reference RNA transcripts, or their protein translation products and a step c) of determining a prognosis response by means of said comparison.

9. The method according to claim 8 wherein determining a prognosis response includes classifying said subject in one of at least two classes corresponding to levels of progression of the disease, preferably said classes are two and corresponds to slow and fast progression of the disease.

10. The method according to anyone of the preceding claims wherein said biological sample is selected from blood, plasma, saliva or urine sample.

11. An *in vitro* method of screening a subject suffering from a neurodegenerative disease for predicting prognosis of the disease comprising at least the step of determining the expression level of at least one RNA transcript in an isolated biological sample from said subject, wherein said RNA transcript is coded by a gene longer or shorter than the median length of coding genes.

12. The method according to any one of the preceding claims wherein said neurodegenerative disease is Parkinson's disease.

13. A kit for performing the method of anyone of claims 1-12, said kit comprising at least means for determining in an isolated biological sample from a subject the expression level of at least one RNA transcript of a gene coding for a product involved in at least one DNA repair process and/or of at least one RNA transcript coded by a gene longer or shorter than the median length of coding genes, preferably further comprising means to normalize the expression level of the RNA transcripts by comparison with the expression level of one or more reference RNA transcripts, or their protein translation products.

14. A computer program comprising instructions which, when the program is executed by a computer, cause the computer to carry out the method of anyone of claims 1-12.

15. A data processing device comprising means adapted for carrying out the method of anyone of claims 1-12, said device preferably comprising:
i) means for receiving data representing the expression level of at least one RNA transcript in an isolated biological sample from a subject, wherein said RNA transcript is the RNA transcript of a gene coding for a product involved in at least one DNA repair process and/or a RNA transcript coded by a gene longer or shorter than the median length of coding genes;
ii) means for receiving data representing at least one reference pattern of RNA transcript expression levels; and
iii) means for comparing said data received by means i) with said data received by means ii) and providing a prognosis of the disease.
